# EUROPEAN PATENT SPECIFICATION

(11) **EP 1 441 650 B1**
(45) Date of publication and mention of the grant of the patent: **31.08.2005**
(21) Application number: 02727940.5
(22) Date of filing: 10.05.2002
(51) Int. Cl.: A61B 17/16

(54) **DRIVE SHAFT COUPLING AND FLEXIBLE SURGICAL REAMER**
ANTRIEBSWELLENKUPPLUNG UND FLEXIBLE CHIRURGISCHE REIBAHLE
ACCOUPLEMENT POUR TIGE D'ENTRAINEMENT ET ALESOIR SOUPLE A USAGE CHIRURGICAL

(30) Priority: 05.11.2001 US 338718 P; 31.01.2002 US 59232
(43) Date of publication of application: 04.08.2004
(73) Proprietor: PRECIMED S.A., 2534 Orvin (CH)
(72) Inventor: WHITE, Patrick, Downingtown, PA 18358 (US)
(74) Representative: Grosfillier, Philippe
(86) International application number: PCT/IB2002/001761
(87) International publication number: WO 2003/039378

(56) References cited:
- EP-A- 0 682 917
- US-A- 5 499 984
- US-A- 5 693 047
- PATENT ABSTRACTS OF JAPAN vol. 1996, no. 02, 29 February 1996 (1996-02-29) & JP 07 265326 A (OLYMPUS OPTICAL CO LTD), 17 October 1995 (1995-10-17)

## Description

### Technical Field

This invention relates generally to torque-transmitting assemblies that have fittings coupled to a flexible drive shaft made of a super-elastic alloy, useful in devices for medical and industrial applications where a flexible shaft is necessary; and in particular to powered surgical instruments for transmitting torque to "flexible reamers" or "flexible drills" to remove material from the center of curved bones during orthopedic surgery.

### Background Art

A torque transmitting assembly according is the preamble of claim 1 is known from US-A-5,499,984.

It is commonly known that nitinol (nickel-titanium) tubing, wire or rod can be used as a mechanical drive shaft. Nitinol is especially useful for transmitting torque while in a bowed or bent shape. These types of drive shafts have proven useful in orthopedic surgical applications where drilling or reaming of curved bones is necessary. One application is to use a drill or reamer with a nitinol drive shaft to clean out the center of a femoral bone before implanting a prosthesis or femoral nail. These bones typically have a bow with a 90-inch radius and require a flexible reamer for the procedure. Nitinol tubing can be used for this application since it is cannulated and can be passed over a guide wire that is placed down the femur before the reaming process begins. Since the tubing is solid it is very easy to clean after the surgical operation since there are no crevices for blood to get trapped in. Earlier designs utilized spring drive shafts and cleaning was extremely difficult since blood could get trapped between the windings of the spring. The earlier spring designs also had difficulties when run in the reverse direction since springs tend to be strong while being used in one direction, however when run in the opposite direction they tend to unwind. To prevent this unwinding problem several manufacturers have added an additional spring inside of the primary spring, which is wound in the opposite direction. Since one spring is inside of the other this contributes to the difficulties with cleaning and further obviates the need for an alternative shaft design.

With the market demand increasing for these novel nitinol drive shafts there have been many attempts to develop safe coupling methods for attachment to the shaft. One difficulty that engineers have been faced with is presented when nitinol tubing exceeds its torsional or fatigue stress limits; it has been known to fail catastrophically and fragment into several sharp pieces. This is dangerous when inside of a patient and poses severe concerns if these types of products are to be used reliably. Historically there have been no solutions offered to limit the stress in the drive shaft, which would eliminate the presently lingering concerns over breakage during use.

Another difficulty is presented with the attachment of the fittings to the nitinol drive shaft. The connection must be reliable and not create any unnecessary stress on the tubing. This will lead to early failure of the shaft. Typically in the orthopedic reamer example mentioned above one end of the nitinol tube has a stainless steel fitting which attaches to a power instrument and on the opposite end either a stainless steel reamer head or an intermediate modular fitting that connects to a reamer head. Several attempts to create reliable attachments have been made.

One approach to the above-mentioned attachment problem has been to use an epoxy to "glue" the fittings onto the nitinol shaft. However, temperatures in the sterilization process and the criticality of surface preparation have rendered this approach unreliable.

Another approach has been to attach the fitting to the shaft with a laser weld; however, the welding process embrittled the tubing and it was known to fail torsional demands in testing. A cross hole and pin were placed through the fittings, however this added approach further proved useless since the matching hole in the tubing created a tremendous stress riser in the tubing causing failure at very low torsional values. In the example mentioned it was known to fail anywhere between 2 to 4 N-M.

Yet another approach has been to press fit the nitinol shaft into a fitting with approximately .002-inch interference. Initial trials worked, however when put through rigorous fatigue tests the tubing placed too much hoop stress on the fittings causing them to fail rather than the shaft. The solution proposed to fix that problem was to add a long section on the fitting that was loosely fit around the tube. This would allow the stress to transition slowly into the area where the press fit was done. This worked successfully, however the solution created a need for the fitting to be extremely long in comparison to the reamer heads being used. This is undesirable since the reamer must follow the curvature of the bone and it did not solve the issue of limiting the torque in the shaft to ensure the safety of the drive shaft during use.

Thus, there remains a primary need to provide a coupling system that is safe and effective for use in surgical and industrial applications where flexible drive shafts are necessary.

There is another need to use a nitinol drive shaft to replace the spring drive shafts in orthopedic instruments and many industrial tools to simplify the cleaning process and ensure consistent torque resistance in the forward and reverse directions.

There is a further need to provide a coupling system which will limit the torque in the nitinol drive shaft fitting to ensure that the coupling limits the torque before the tubing breaks.

There is yet a need for a coupling that will not place any unnecessary stress on the nitinol tubing causing it to prematurely break.

There is also a need to shorten the length of the fitting so that the reamer can follow the natural contour of the inside of the bone while transferring the stress smoothly so as to ensure the strength of the fitting.

### Disclosure of Invention

According to the invention, a torque-transmitting assembly is described in claim 1, as well as a method of forming the assembly in claim 21 and a surgical reamer that includes the assembly (claim 25). The assembly has a female coupling member with a bore. The female coupling member may be a fitting that connects to a power instrument or it may present a cutting head, or both. A radially flexible member is received within the bore, defining a hollow shape with an opening. The assembly also has an elongated shaft member made of a super-elastic alloy, received within the opening. Relative motion among the members causes the radially flexible member to contact the shaft, inducing a super-elastic activation in the shaft that urges the shaft and radially flexible member into surface-to-surface contact, securing the members together in a fixed relative position. In a preferred embodiment, the radially flexible member has an external surface that frictionally engages the bore upon relative motion. The contact still preferably occurs along one or more areas that frictionally carry the applied torque, which contact area may be calibrated so that the contact slips at a preset torque before the failure strength of the shaft is reached. In another preferred embodiment, the shaft is tubular with a cannulation, which may further be aligned with another cannulation in the female coupling member for common passage of a guide wire. In yet another preferred embodiment, an inter-positional polymer sleeve is provided in the assembly for transmitting bending stress. In a still another preferred embodiment, the female coupling member has a counter-bore, while the radially flexible member has an exterior surface adapted for engagement within the counter-bore and may be compressed within the counter-bore or be in a pre-assembled state therein. In one alternative, preferred embodiment, the radially flexible member has a split collet, whereupon relative motion among at least two of the members causes the opening to contact the shaft, inducing a super-elastic activation in the shaft that urges the shaft and the collet into surface-to-surface contact, securing the members together in a fixed relative position. In another alternative, preferred embodiment, the radially flexible member is a collar and made of super-elastic alloy, whereupon relative motion between the fitting and the collar causes the collar to contact the shaft, inducing a super-elastic activation in the shaft that engages the shaft and collar into surface-to-surface contact, securing the members together in a fixed relative position. The collar may further be a washer or a series of washers.

A method of forming a torque-transmitting assembly is also disclosed, having the following steps. A female coupling member is provided, with a bore, as is a radially flexible member with an external surface and an opening, the radially flexible member being situated within the bore. An elongated shaft member is provided, made of a super-elastic alloy, and is received within the opening. Relatively moving at least two of the members, causes the radially flexible member to contact the shaft, inducing a super-elastic activation in the shaft that urges the shaft and radially flexible member into surface-to-surface contact, securing the members together in a fixed relative position. The radially flexible member may alternatively consist of either a split collet operable with an outer compression sleeve, or a super-elastic collar operable within a counter-bore of the female coupling.

A flexible surgical reamer having a torque-transmitting assembly is also disclosed.

An advantage of the present invention is a torque-transmitting coupling assembly that is also torque-limiting, relying upon a surface-to-surface frictional contact, thus slippage of the coupling occurs at torque less than the maximum failure strength of the shaft.

Another advantage of the present invention is that the torque can be adjusted, by increasing or decreasing the area of contact in interference and adjusting the surface finishes on the assembly components.

A further advantage is the amelioration of fatigue due to hoop stress by use of a preferred softer material, such a polymer sleeve interposed between the coupling member and the shaft-- this acts as a dampener to spread out the forces smoothly ensuring the proper stress transfer.

Other objects and advantages of the invention will become apparent upon reading the following Detailed Description and upon reference to the appended Drawings.

### Brief Description of Drawings

**FIG. 1** is an exploded view of a torque-transmitting assembly of the present invention, showing a preferred fitting formed with a split collet, a compression sleeve and an optional polymer sleeve interposed therebetween;
**FIG. 2** is an elevational view of **FIG. 1,** showing the collet compressed by the sleeve to couple the assembly;
**FIG. 3** is a sectional view taken substantially along the Lines **3-3** of **FIG. 2**;
**FIG. 4** is an exploded view of another embodiment of a torque-transmitting assembly of the present invention, showing a fitting with a collar preferably having a series of washers each made of super-elastic alloy, a compression sleeve and an optional polymer sleeve;
**FIG. 5** is an elevational view of **FIG. 4**, showing the collar compressed by the sleeve to couple the assembly; and
**FIG. 6** is sectional view taken substantially along the Lines **6-6** of **FIG. 5**.

### Best Mode for Carrying Out the Invention

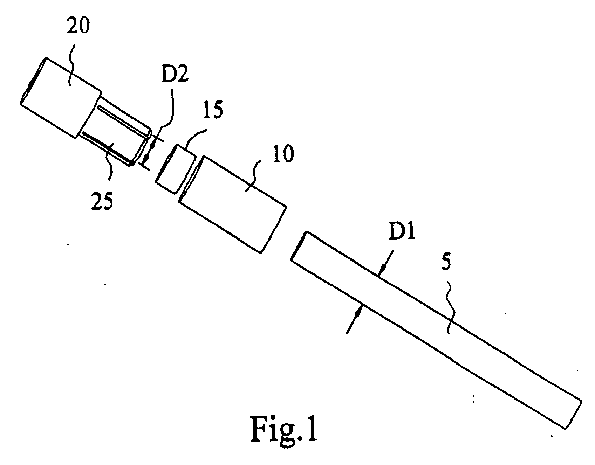

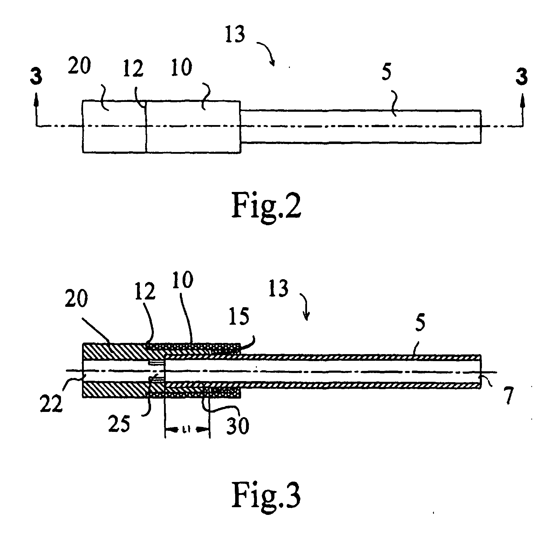

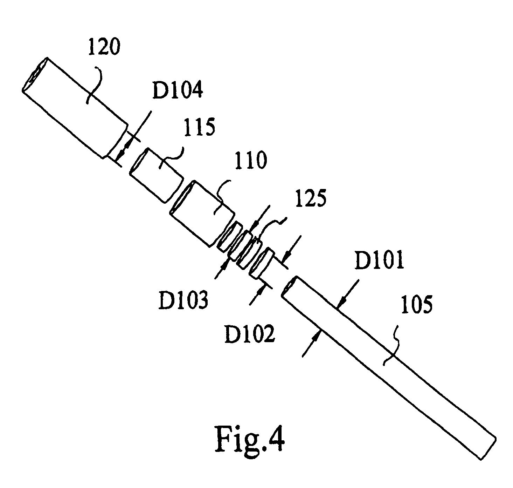

Referring to **FIGS. 1-3** and particularly **FIG. 1,** the present invention includes a drive shaft **5** made of a super-elastic alloy, preferably a nickel-titanium commonly known as nitinol. Shafts made from this type of alloy can be formed with a cannulation **7** as shown in **FIG. 3** and exhibit the distinctive characteristic of transferring torque while subjected to high bending forces during use. The exemplary use of the present invention is in orthopedic drilling and reaming devices; however, the usage of super-elastic alloys according to the invention has much broader applications encompassing both medical as well as other industrial applications. A device of the present invention is generally shown at **13** in **FIGS. 2-3.** Tool fitting **20** preferably has a cannulation **22** that is aligned with another cannulation **7** formed in shaft **5** when device **13** is assembled according to the present method. The aligned cannulations **7, 20** allow device **13** to be placed over the top of a guide wire (not shown). Tool fitting **20** also has a radially moveable, i.e., flexible collet portion **25**, which is preferably an integral structure although the fitting may be separate from the radially flexible portion as discussed below in conjunction with another embodiment (**FIGS. 4-6**). According to the present method, shaft **5** is slid into collet **25.** Preferably there is an interference fit between the outer diameter **D1** of shaft **5** and the inner diameter **D2** of collet **25.** This interference causes collet **25** to bend out in a flower configuration as shaft **5** and fitting **20**, are slid together.

Once shaft **5** has been slid into collet **25**, a compression sleeve **10** (**FIGS. 1-3**) is slid over the flower shaped collet **25** and welded at junction **12**. As these components **5**, **10**, **25** are assembled, the collet **25** is forced radially onto the shaft causing a super-elastic activation of the alloy forming the shaft to thereby effect a secure coupling. This super-elastic reaction allows the fingers of collet **25** to contact the tube along the length **L1** as shown in **FIG. 3.** This surface-to-surface contact (shown at **30** in **FIG. 3**) allows the device **13** to transmit torque. In essence the components are transmitting torque via friction. Thus, whenever the frictional forces are overcome by application of too much torque to the tool fitting **20**, the fitting and shaft **5** break free of one another to slip rotationally. This slippage limits the amount of torque that can be applied to the shaft. The contact surface **30** can be adjusted by design to change the length **L1,** in turn, adjusting the maximum applicable torque limit to ensure that the slippage occurs before the maximum yield strength is reached in shaft **5**.

Compression sleeve **10** may have an optional polymer sleeve **15** pre-assembled inside. The purpose of polymer sleeve **15** is to transfer stress through tool fitting **20** in a uniform manner to shaft **5** during its use in a bent configuration. This smoother transition ensures premature failure of fitting **20**. Use of polymer sleeve **15** may be found necessary when the design of the wall thickness of tool fitting **20** becomes thin, e.g., between about .25 mm to 1.00 mm. Otherwise, tool fitting **20** will be able to handle the stress eliminating the necessity for polymer sleeve **15**.

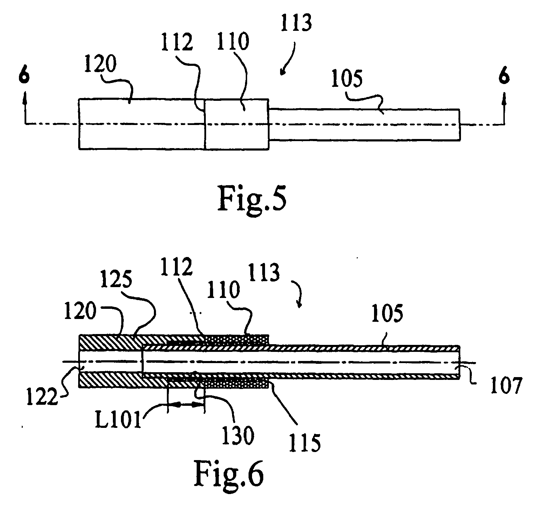

Another preferred embodiment of the present invention is depicted in **FIGS. 4-6**, particularly **FIG. 4**, which shows a shaft **105** made of super-elastic alloy such as nitinol. As discussed relative to **FIGS. 1-3,** shaft **105** is similar, preferably having a cannulation **107** as shown in **FIG. 6**. Shaft **105** thus can exhibit the distinctive characteristic of transferring torque while exposed to high bending forces during use. Although the exemplary uses of super-elastic alloy herein are orthopedic drilling and reaming devices, use of this alloy in the present invention has much broader applications encompassing both medical as well as other industrial applications. The assembled device is generally shown at **113** in **FIGS. 5-6** and utilizes a tool fitting **120** that is modular rather than a unitary component (see collet **25** in **FIGS. 1-3**). Tool fitting **120** may have a cannulation **122,** which is useful while aligned with the shaft cannulation **107** to allow it to be placed over the top of a guide wire (not shown). Tool fitting **120** is sized with inner diameter **D104** and has a modular radially flexible portion comprising a series of nitinol washers **125** that together are collar-shaped. Washers **125** each have an outer diameter **D103** and an inner diameter **D102**. During assembly according to another, preferred method of the present invention, shaft **105** having diameter **D101** is slid into the tool fitting **120**. Preferably upon assembly there is an interference fit between the outer diameter **D101** and the inner diameter **D102** and an interference fit between the outer diameter **D103** and **D104**. This interference causes a radial compression in the flexible washers **125** causing surface-to-surface contact as shown at **130** along length **L101**. The radially flexible washers **125** end up interposed between the shaft **105** and the tool fitting **120**. This can be accomplished through various assembly methods. In one instance the flexible washers **125** can be pre-assembled with the tool fitting **120** and then the shaft **105** can subsequently be introduced to the assembly. Preferably though, the shaft **105** can be loosely placed in the tool fitting **120** and then the washers can be advanced into the tool fitting **120**. As they are slid into place they compress via interference between the outer dimension **D103** of the washers **125** and the inner dimension **D104** of the tool fitting. This interference causes a super-elastic reaction in the washers **125** causing their inner dimension **D102** to compress against the shaft's **105** outer dimension **D101** causing the surface-to-surface contact as shown at **130** along length **L101.** It is this surface to surface contact shown at **130** in **FIG. 6** that allows the assembly to transmit torque. In essence the components are transmitting torque-using friction. If the frictional forces are overcome by applying too much torque to the tool fitting **120** the components **120,105** break free and slip rotationally. This rotational slip limits the amount of torque that can be applied to the shaft. If the contact surface **130** is adjusted during design by changing the length **L101** the maximum applicable torque limit can be adjusted to ensure that the slippage occurs before the maximum yield strength is reached in the shaft **105.**

An additional sleeve **110** can be added and welded at junction **112** with an optional polymer sleeve **115** pre-assembled inside. The purpose of the polymer sleeve is to transfer stress through the tool fitting **120** in a uniform manner to the shaft **105** during use in a bent configuration. This smoother transition ensures premature failure of the fitting. The use of this polymer sleeve **115** may only be necessary when the design of the wall thickness of the tool fitting **120** becomes thin, somewhere on the order of .25 mm to 1mm. Other wise the tool fitting **120** will be able to handle the stress eliminating the necessity for the additional polymer sleeve **115** and sleeve **110**.

The following documents are also referred to US Patent application Ser. No. 09/860,916, filed May 18, 2001 and entitled, "Stress-Induced Connecting Assembly"; U.S. Patent No. 6,257,953, filed March 11, 2000 and entitled, "Stress-Induced Interposed Connector"; US Patent Application Ser. No. 09/311,938, filed May 14, 1999 and entitled "Stress-Induced Seal"; and US Provisional Patent Application Ser. No. 60/262,362, filed January 19, 2001 and entitled "Drive Shaft Coupling".

Although the invention has been described with reference to preferred embodiments thereof, it is evident to those of skill in the art that various modifications and improvements may be made without departing from the scope of the invention as defined by the appended claims.

## Claims

1. A torque-transmitting assembly (13, 113) comprising:
a) a female coupling member (10; 120) with a bore and an elongated shaft member, **characterized in that**
b) a radially flexible member (20, 25; 125), received within the bore, defining a hollow shape with an opening; and
c) the elongated shaft member (5; 105) is made of a super-elastic alloy, received within the opening,
wherein the members are secured together in a fixed relative position by
relative motion among at least two of the members which causes the radially flexible member (20, 25; 125) to contact the shaft member (5; 105), inducing a super-elastic activation in the shaft member that urges the shaft member and radially flexible member (20, 25; 125) into surface-to-surface contact.

2. The assembly according to Claim 1 wherein the radially flexible member (20, 25; 125) has an external surface that frictionally engages the bore upon relative motion.

3. The assembly according to one of the Claims 1 or 2 wherein the shaft (5; 105) is tubular with a cannulation (7; 107).

4. The assembly of Claim 3 wherein the bore of the female coupling member (10; 120) further comprises a cannulation (122) aligned with the shaft member cannulation (7; 107), for common passage of a guide wire therethrough.

5. The assembly according to one of the Claims 1 to 4 further comprising an inter-positional polymer sleeve (15; 115) for transmitting bending stress in the assembly.

6. The assembly according to one of the Claims 1 to 5 wherein the contact occurs in one or more areas (L1; L101) that frictionally carries the applied torque.

7. The assembly according to Claim 6 wherein the contact area (L1; L101) is calibrated so that the contact slips at a preset torque before the failure strength of the shaft member (5; 105) is reached.

8. The assembly according to one of the Claims 1 to 7 wherein the female coupling member (120) further comprises a counter-bore and the radially flexible member (125) has an exterior surface adapted for engagement within the counter-bore.

9. The assembly according to Claim 8 wherein the radially flexible member (125) is compressed within the counter-bore.

10. The assembly according to one of the Claims 1 to 9 wherein the female coupling member (120)) is a fitting that connects the assembly to a cutting tool-bit or powered instrument.

11. The assembly according to one of the Claims 1 to 9 wherein the female coupling member (120) further comprises a fitting with a cutting tool-bit.

12. The assembly according to Claim 11 wherein the assembly is further connected to a powered instrument.

13. The assembly according to one of the Claims 1 to 7 wherein the radially flexible member (20, 25) is a split collet (25).

14. The assembly according to one of the Claims 1 to 12, wherein the radially flexible member (125) is in the form of a collar and made of super-elastic alloy and the relative motion further induces a super-elastic activation of the collar (125).

15. The assembly according to one of the Claims 1 to 12 or 14 wherein the radially flexible member is a washer (125).

16. The assembly according to one of the Claims 1 to 12 or 14 wherein the radially flexible member comprises a series of washers.

17. The assembly according to Claim 14 wherein the super-elastic alloy is a nickel-titanium alloy.

18. The assembly according to Claim 13 wherein the split collet (25)is connected to a cutting tool-bit or powered instrument.

19. The assembly according to Claim 18 wherein the split collet (25) further comprises a cutting tool-bit.

20. The assembly according to Claim 19 further coupled to a powered instrument.

21. A method of forming a torque-transmitting assembly (13, 113), comprising the steps of:
a) providing a female coupling member (10; 120) with a bore;
b) providing a radially flexible member (20, 25; 125) with an external surface and an opening, situating the radially flexible member within the bore
c) providing an elongated shaft member(5; 105 made of a super-elastic alloy, received within the opening; and
d) relatively moving at least two of the members, causing the radially flexible member to contact the shaft member (5, 105), inducing a super-elastic activation in the shaft member that urges the shaft member and radially flexible member (20, 25; 125)into surface-to-surface contact, securing the members together in a fixed relative position.

22. The method according to Claim 21 wherein step d) further comprises frictionally engaging the members along a contact area (L1; L101) that carries the applied torque, the contact area being calibrated to slip at a preset torque before the failure strength of the shaft member (5; 105) is reached.

23. The method according to one of the Claims 21 or 22 further comprising the steps of providing the female coupling member (120) with a counter-bore, providing the radially flexible member (125) in the form of a collar made of super-elastic alloy and inducing a super-elastic activation in the collar.

24. The method according to Claim 22 wherein step a) further comprises providing a radially flexible member in the form of a split collet (25)

25. A flexible surgical reamer having a torque-transmitting assembly according to one of the claims 1 to 20.

## Patentansprüche

1. Drehmomentübertragende Anordnung (13, 113), enthaltend:
a) ein aufnehmendes Kupplungsteil (10; 120) mit einer Bohrung und einem länglichen Schaftelement, **dadurch gekennzeichnet, dass**
b) ein radial nachgiebiges Element (20, 25; 125), welches in die Bohrung eingesetzt ist, eine Hohlform mit einer Öffnung definiert; und
c) das längliche Schaftelement (5; 105), das aus einer superelastischen Legierung besteht, in die Öffnung eingeführt ist, wobei die Elemente in einer festen gegenseitigen Position infolge einer Relativbewegung von mindestens zwei der obigen Elemente gesichert sind, und wobei diese Relativbewegung das radial nachgiebige Element (20, 25; 125) dazu veranlasst, in Berührung mit dem Schaftelement (5; 105) zu gelangen, wodurch eine superelastische Aktivierung am Schaftelement eingeleitet wird, welche das Schaftelement in Oberflächenkontakt mit dem radial nachgiebigen Element (20, 25; 125) bringt.

2. Anordnung nach Anspruch 1, bei der das radial nachgiebige Element (20, 25; 125) eine Aussenfläche aufweist, die sich bei einer Relativbewegung durch Reibung an die Bohrung anlegt.

3. Anordnung nach Anspruch 1 oder 2, bei der der Schaft (5; 105) rohrförmig mit einer Kanüle (7; 107) ausgebildet ist.

4. Anordnung nach Anspruch 3, bei der die Bohrung des aufnehmenden Kupplungsteils (10; 120) weiterhin eine Kanüle (122) aufweist, die zwecks gemeinsamem Durchgang eines Führungsdrahtes mit der Kanüle (7; 107) des Schaftelementes ausgerichtet ist.

5. Anordnung nach einem der Ansprüche 1 bis 4, welche weiterhin eine Polymerhülse (15; 115) als Zwischenlage zur Übertragung einer Biegebeanspruchung der Anordnung aufweist.

6. Anordnung nach einem der Ansprüche 1 bis 5, bei der die Berührung an einem oder mehreren Flächenbereichen (L1; L101) stattfindet, welche das angelegte Drehmoment durch Reibung aufnehmen.

7. Anordnung nach Anspruch 6, bei der die Berührungsfläche (L1; L101) derart kalibriert ist, dass die Berührung bei einem vorgegebenen Drehmoment einen Schlupf aufweist, bevor die Grenzfestigkeit des Schaftelementes (5; 105) erreicht ist.

8. Anordnung nach einem der Ansprüche 1 bis 7, bei der das aufnehmende Kupplungselement (120) weiterhin eine Gegenbohrung aufweist und das radial nachgiebige Element (125) eine Aussenfläche besitzt, die zum Eingriff in die Gegenbohrung ausgebildet ist.

9. Anordnung nach Anspruch 8, bei der das radial nachgiebige Element (125) in der Gegenbohrung komprimiert wird.

10. Anordnung nach einem der Ansprüche 1 bis 9, bei der das aufnehmende Kupplungsteil (120) eine Muffe ist, welche die Anordnung mit einem Schneidwerkzeugeinsatz oder einem angetriebenen Instrument verbindet.

11. Anordnung nach einem der Ansprüche 1 bis 9, bei der das nachgiebige Kupplungsteil (120) weiterhin eine Anschlusshülse mit einem Schneidwerkzeugeinsatz aufweist.

12. Anordnung nach Anspruch 11, bei der die Anordnung weiterhin mit einem angetriebenen Instrument verbunden ist.

13. Anordnung nach einem der Ansprüche 1 bis 7, bei der das radial nachgiebige Element (20, 25) eine Schlitzhülse (25) ist.

14. Anordnung nach einem der Ansprüche 1 bis 12, bei der das radial nachgiebige Element (125) in Form einer Hülse vorliegt und aus einer superelastischen Legierung hergestellt ist, und dass die Relativbewegung weiterhin eine superelastische Aktivierung der Hülse (125) verursacht.

15. Anordnung nach einem der Ansprüche 1 bis 12 oder 14, bei der das radial nachgiebige Element eine Zwischenscheibe (125) ist.

16. Anordnung nach einem der Ansprüche 1 bis 12 oder 14, bei der das radial nachgiebige Element eine Reihe von Zwischenscheiben darstellt.

17. Anordnung nach Anspruch 14, bei der die superelastische Legierung eine Nickel-Titan-Legierung ist.

18. Anordnung nach Anspruch 13, bei der die Schlitzhülse (25) mit einem Schneidwerkzeugeinsatz oder einem motorgetriebenen Instrument verbunden ist.

19. Anordnung nach Anspruch 18, bei der die Schlitzhülse (25) weiterhin einen Schneidwerkzeugeinsatz aufweist.

20. Anordnung nach Anspruch 19, die weiterhin an ein angetriebenes Instrument angeschlossen ist.

21. Verfahren zur Erstellung einer drehmomentübertragenden Anordnung (13, 113), enthaltend die folgenden Schritte:
a) Bereitstellung eines aufnehmenden Kupplungsteils (10; 120) mit einer Bohrung;
b) Bereitstellung eines radial nachgiebigen Elements (20, 25; 125) mit einer Aussenfläche und einer Öffnung, Einsetzen des radial nachgiebigen Elements in die Bohrung;
c) Bereitstellung eines länglichen Schaftelements (5; 105) aus einer Superlegierung, welches sich in der Öffnung befindet; und
d) Ausführung einer Relativbewegung von mindestens zwei der obigen Elemente, wodurch das radial nachgiebige Element in Berührung mit dem Schaftelement (5; 105) gelangt, Einleiten einer superelastischen Aktivierung im Schaftelement, welche die Oberfläche des Schaftelements und des radial nachgiebigen Elements (20, 25; 125) in Kontakt bringt, so dass die Elemente in einer festen gegenseitigen Stellung gesichert werden.

22. Verfahren nach Anspruch 21, bei dem der Schritt d) weiterhin eine Reibungsverbindung der Elemente entlang einer Berührungsfläche (L1; L101) umfasst, welche das angelegte Drehmoment aufnimmt, wobei die Berührungsfläche derart kalibriert ist, dass bei einem vorbestimmten Drehmoment ein Schlupf eintritt, bevor die Grenzfestigkeit des Schaftelements (5; 105) erreicht ist.

23. Verfahren nach Anspruch 21 oder 22, bei dem weiterhin das aufnehmende Kupplungsteil (120) mit einer Gegenbohrung versehen wird, dass das radial nachgiebige Element (125) als Hülse aus einer superelastischen Legierung gefertigt wird, und dass eine superelastische Aktivierung in der Hülse herbeigeführt wird.

24. Verfahren nach Anspruch 22, bei dem in Schritt a) weiterhin ein radial nachgiebiges Element in Form einer Schlitzhülse (25) vorgesehen wird.

25. Flexible chirurgische Reibahle mit einer drehmomentübertragenden Anordnung nach einem der Ansprüche 1 bis 20.

## Revendications

1. Assemblage pour transmettre un couple (13, 113), comprenant:
a) un élément d'accouplement femelle (10; 120) comprenant un alésage et un élément de tige allongé, **caractérisé en ce que**
b) un élément radialement flexible (20, 25; 125), reçu dans l'alésage, définissant une forme creuse ayant une ouverture; et
c) l'élément de tige allongé (5; 105) est fabriqué à partir d'un alliage super-élastique et reçu dans l'ouverture,
dans lequel les éléments sont bloqués les uns aux autres dans une position relative fixe par un mouvement relatif d'au moins deux éléments qui amène l'élément radialement flexible (20, 25; 125) à entrer en contact avec l'élément de tige (5; 105) induisant une activation super-élastique dans l'élément de tige qui pousse l'élément de tige et l'élément radialement flexible (20, 25; 125) dans un contact de surface à surface.

2. Assemblage selon la revendication 1, dans lequel l'élément radialement flexible (20, 25; 125) présente une surface extérieure qui forme un contact de friction avec l'alésage lors d'un mouvement relatif.

3. Assemblage selon la revendication 1 ou 2, dans lequel la tige (5; 105) forme un tube comportant une canule (7; 107).

4. Assemblage selon la revendication 3, dans lequel l'alésage de l'élément d'accouplement femelle (10; 120) comprend en plus une canule (122) alignée à la canule de l'élément de tige (7; 107) pour le passage commun d'un fil de guidage.

5. Assemblage selon l'une des revendications 1 à 4, comprenant en plus une douille en polymère interpositionnelle (15; 115) pour transmettre une charge de flexion à l'assemblage.

6. Assemblage selon l'une des revendications 1 à 5, dans lequel le contact se forme dans une ou plusieurs aires (L1; L101) qui transmettent par friction le couple appliqué.

7. Assemblage selon la revendication 6, dans lequel l'aire de contact (L1; L101) est calibrée de telle façon que le contact commence à glisser à un couple préétabli avant d'atteindre la charge de défaillance de l'élément de tige (5; 105).

8. Assemblage selon l'une des revendications 1 à 7, dans lequel l'élément d'accouplement femelle (120) comprend en plus un contre-alésage, et que l'élément radialement flexible (125) présente une surface extérieure adaptée à l'engagement dans le contre-alésage.

9. Assemblage selon la revendication 8, dans lequel l'élément radialement flexible (125) est comprimé à l'intérieur du contre-alésage.

10. Assemblage selon l'une des revendications 1 à 9, dans lequel l'élément d'accouplement femelle (120) est un raccord qui connecte l'assemblage à une partie d'outil de coupe ou à un instrument motorisé.

11. Assemblage selon l'une des revendications 1 à 9, dans lequel l'élément d'accouplement femelle (120) comprend en plus un raccord avec une partie d'outil de coupe.

12. Assemblage selon la revendication 11, dans lequel l'assemblage est en plus connecté à un instrument motorisé.

13. Assemblage selon l'une des revendications 1 à 7, dans lequel l'élément radialement flexible (20, 25) est un manchon fendu (25).

14. Assemblage selon l'une des revendications 1 à 12, dans lequel l'élément radialement flexible (125) présente la forme d'un collet et est fabriqué à partir d'un alliage super-élastique, et que le mouvement relatif induit en plus une activation super-élastique du collet (125).

15. Assemblage selon l'une des revendications 1 à 12 ou 14, dans lequel l'élément radialement flexible est un disque d'espacement (125).

16. Assemblage selon l'une des revendications 1 à 12 ou 14, dans lequel l'élément radialement flexible comprend plusieurs disques d'espacement.

17. Assemblage selon la revendication 14, dans lequel l'alliage super-élastique est un alliage de nickel et de titane.

18. Assemblage selon la revendication 13, dans lequel le manchon fendu (25) est connecté à une partie d'outil de coupe ou à un instrument motorisé.

19. Assemblage selon la revendication 18, dans lequel le manchon fendu (25) comprend en plus une partie d'outil de coupe.

20. Assemblage selon la revendication 19, qui est en plus accouplé à un instrument motorisé.

21. Procédé pour former un assemblage (13, 113) apte à transmettre un couple, comprenant les étapes suivantes:
a) la fourniture d'un élément d'accouplement femelle (10; 120) comportant un alésage;
b) la fourniture d'un élément radialement flexible (20, 25; 125) ayant une surface extérieure et une ouverture, et l'introduction de l'élément radialement flexible dans l'alésage;
c) la fourniture d'un élément de tige allongé (5; 105) fabriqué à partir d'un alliage super-élastique et reçu dans l'ouverture; et
d) le mouvement relatif d'au moins deux des éléments, provoquant le contact de l'élément radialement flexible avec l'élément de tige (5; 105), induisant une activation super-élastique dans l'élément de tige qui pousse l'élément de tige et l'élément radialement flexible (20, 25; 125) dans un contact de surface à surface, bloquant les éléments les uns aux autres dans une position relative fixe.

22. Procédé selon la revendication 21, dans lequel l'étape d) comprend en plus l'engagement par friction des éléments le long d'une aire de contact (L1; L101) qui porte le couple appliqué, l'aire de contact étant calibrée pour glisser à un couple préétabli avant d'atteindre la charge de défaillance de l'élément de tige (5; 105).

23. Procédé selon la revendication 21 ou 22, comprenant en plus les étapes d'équiper l'élément d'accouplement femelle (120) d'un contre-alésage, de fournir l'élément radialement flexible (125) en forme de collet fabriqué à partir d'un alliage super-élastique et d'induire une activation super-élastique dans le collet.

24. Procédé selon la revendication 22, dans lequel l'étape a) comprend en plus la fourniture de l'élément radialement flexible en forme de manchon fendu (25).

25. Alésoir chirurgical souple, comportant un assemblage apte à transmettre un couple selon l'une des revendications 1 à 20.
